Europäisches Patentamt

European Patent Office

Office européen des brevets

(11) Veröffentlichungsnummer: **0 001 211**
A1

(12) # EUROPÄISCHE PATENTANMELDUNG

(21) Anmeldenummer: **78100660.6**

(22) Anmeldetag: **14.08.78**

(51) Int. Cl.²: **C 07 H 3/04**
C 07 H 3/02, C 07 C 49/17
C 07 C 47/19, C 07 C 47/10
C 07 C 31/18, C 08 G 18/32
C 08 G 63/12, C 08 G 65/28
//C08G18/42, C08G18/48

(30) Priorität: **25.08.77 DE 2738274**

(43) Veröffentlichungstag der Anmeldung:
**04.04.79 Patentblatt 79/7**

(84) Benannte Vertragsstaaten:
**BE DE FR GB NL**

(71) Anmelder: **Bayer Aktiengesellschaft**
**Zentralbereich Patente,Marken und Lizenzen Bayerwerk**
**D-5090 Leverkusen 1(DE)**

(72) Erfinder: **Möhring, Edgar, Dr.**
**Huferweg 49a**
**D-5060 Bergisch-Gladbach 2(DE)**

(72) Erfinder: **Müller, Hanns Peter, Dr.**
**Berta-von-Suttner-Strasse 40**
**D-5090 Leverkusen 1(DE)**

(54) **Verfahren zur Entfernung von Bleiionen aus Formose und Verwendung der so erhaltenen Formose zur Herstellung von Kunststoff und metholierter Formosen.**

(57) Die vorliegende Erfindung betrifft ein kontinuierliches oder diskontinuierliches Verfahren zur Entfernung katalytischer Mengen von Bleiionen aus wäßrigen Formoselösungen durch elektrochemische, kathodische Abscheidung.

EP 0 001 211 A1

- 1 -

BAYER AKTIENGESELLSCHAFT  5090 Leverkusen, Bayerwerk
Zentralbereich  Sft/bc **BEZEICHNUNG GEÄNDERT**
Patente, Marken und Lizenzen  **siehe Titelseite**

Verfahren zur Entfernung von Bleiionen aus Formose

Die vorliegende Erfindung betrifft ein kontinuierliches oder diskontinuierliches Verfahren zur Entfernung katalytischer Mengen von Bleiionen aus wäßrigen Formoselösungen durch elektrochemische, kathodische Abscheidung.

Unter "Formose" werden erfindungsgemäß die an sich bekannten Gemische von niedermolekularen Polyhydroxyl-Verbindungen (mehrwertigen Alkoholen, Hydroxyaldehyden und Hydroxyketonen) verstanden, welche bei der Kondensation von Formaldehydhydrat entstehen.

Die Herstellung von Gemischen mehrwertiger Alkohole, Hydroxyaldehyde und Hydroxyketone durch Selbstkondensation des Formaldehydhydrats wird in zahlreichen Literaturstellen beschrieben. Beispielsweise seien in diesem Zusammenhang Butlerow und Loew, Annalen 120, 295 (1861), bzw. J.pr.Chem. 33, 321 (1886), Pfeil, chemische Berichte 84, 229 (1951), Pfeil und Schroth, chemische Berichte 85, 303 (1952), R.D. Partridge und A.H. Weiss, Carbohydrate Research 24,

Le A 18 372 - Ausland

29-44 (1972), die Formosen aus Glycerinaldehyd bzw. Dioxyaceton nach Emil Fischer, die deutschen Patentschriften 822 385, 830 951 und 884 794, die US-Patentschriften 2 224 910, 2 269 935 und 2 272 378 sowie die englische Patentschrift 513 708 genannt. Diese bekannten Verfahren des Standes der Technik sind mit gewissen Nachteilen behaftet (schlechte Raum/Zeit-Ausbeuten, gefärbte Nebenprodukte); in jüngster Zeit wurden jedoch von der Anmelderin noch nicht veröffentlichte neue Verfahren entwickelt, nach denen sich in hoher Ausbeute mit gängigen Katalysatoren praktisch farblose und von störenden Nebenprodukten freie Formosen herstellen lassen.

Eines dieser neuen Verfahren besteht darin, daß man die Kondensation des Formaldehydhydrats in Gegenwart von löslichen oder unlöslichen Blei(II)salzen, bzw. an hochmolekulare Träger gebundenen Blei(II)ionen als Katalysator und eines Gemisches aus Hydroxyaldehyden und Hydroxyketonen als Co-Katalysator ablaufen läßt, wie es bei der Kondensation von Formaldehydhydrat entsteht und welches durch folgende Molverhältnisse charakterisiert ist:

Verbindungen mit 3 C-Atomen/Verbindungen mit 4 C-Atomen:
0,5 bis 2,0
Verbindungen mit 4 C-Atomen/Verbindungen mit 5 C-Atomen:
0,2 bis 2,0
Verbindungen mit 5 C-Atomen/Verbindungen mit 6 C-Atomen:
0,5 bis 5,0
wobei der Anteil der Komponenten mit 3 bis 6 C-Atomen mindestens 75 Gew.-%, vorzugsweise mehr als 85 Gew.-%, bezogen auf gesamten Co-Katalysator, beträgt.

Die Reaktionstemperatur liegt dabei im allgemeinen zwischen 70 und 110°C, bevorzugt zwischen 80 und 100°C, und

Le A 18 372

der pH-Wert der Reaktionslösung wird durch kontrollierte Zugabe einer anorganischen oder organischen Base bis zu einem Umsatz von 10-60 %, vorzugsweise 30-50 %, auf einen Wert von 6,0-8,0, bevorzugt 6,5-7,0 und anschließend auf einen Wert von 4,0-6,0, bevorzugt 5,0-6,0 eingestellt. Überraschenderweise läßt sich die Produktverteilung der entsprechenden Polyol-, Hydroxyaldehyd- und Hydroxyketongemische durch diese spezielle pH-Führung und durch anschließende Kühlung bei verschieden hohen Restformaldehydgehalten (0 bis 10 Gew.-%, vorzugsweise 0,5 bis 6 Gew.-%) in reproduzierbarer Weise variieren.

Nachdem man die Selbstkondensation des Formaldehydhydrats durch Kühlen und/oder durch Desaktivierung des bleihaltigen Katalysators mittels Säuren unterbrochen hat, wird der Katalysator entfernt und gegebenenfalls das in den Produkten enthaltene Wasser destillativ entfernt.

Eine weitere Möglichkeit, in hoher Raum-Zeit-Ausbeute hochkonzentrierte farblose Formosen herzustellen, besteht darin, wäßrige Formalinlösungen und/oder Paraformaldehyd-Dispersionen in Gegenwart eines löslichen oder unlöslichen Metallkatalysators und eines durch teilweise Oxidation eines zwei- oder mehrwertigen, mindestens zwei benachbarte Hydroxylgruppen aufweisenden Alkohols mit einem Molekulargewicht zwischen 62 und 242 bzw. eines Gemisches derartiger Alkohole dargestellten Co-Katalysators zu kondensieren, wobei man den pH-Wert der Reaktionslösung durch gesteuerte Zufuhr einer Base bis zu einem Umsatz von 5 bis 40 % zwischen 6,0 und 9,0 hält und anschließend bis zum Abbruch der Kondensations-

Le A 18 372

- 4 -

reaktion auf 4,5 bis 8,0 einstellt, so daß er nun um 1,0 bis 2,0 Einheiten tiefer liegt als in der ersten Reaktionsphase, dann die Reaktion bei einem Restgehalt von 0-10 Gew.-% Formaldehyd durch Desaktivierung des Katalysators unterbricht und den Katalysator entfernt.

Qualitativ hochwertige Formosen können auch durch Kondensation von Formaldehyd in Gegenwart eines Metallkatalysators und mehr als 10 Gew.-%, bezogen auf Formaldehyd, eines oder mehrerer zwei- oder mehrwertiger niedermolekularer Alkohole und/oder höhermolekularer Polyhydroxylverbindungen hergestellt werden.

Besonders wirtschaftlich ist es, gemäß einem weiteren noch unveröffentlichten Verfahren der Anmelderin , Formose direkt, d.h. ohne den Umweg über wäßrige Formalinlösungen oder Paraformaldehyd, aus Formaldehyd enthaltenden Synthesegasen herzustellen.

Man leitet zu diesem Zweck die Synthesegase, wie sie bei der großtechnischen Herstellung von Formaldehyd anfallen, bei Temperaturen zwischen 10 und 150°C kontinuierlich oder diskontinuierlich in eine Absorptionsflüssigkeit, welche aus Wasser, ein- oder mehrwertigen niedermolekularen Alkoholen und/oder höhermolekularen Polyhydroxylverbindungen und/oder zur Endiolbildung befähigten Verbindungen als Co-Katalysator und/oder löslichen oder unlöslichen Metallverbindungen, welche gegebenenfalls an hochmolekulare Träger gebunden sind, als Katalysator besteht und einen pH-Wert von 3 bis 10 aufweist, kondensiert den Formaldehyd direkt in situ in der Absorptionsflüssigkeit (gegebenenfalls auch in einem

Le A 18 372

- 5 -

nachgeschalteten Reaktionsrohr bzw. einer nachgeschalteten Rührkesselkaskade), bricht die Selbstkondensation des Formaldehyds bei einem Restformaldehydgehalt im Reaktionsgemisch von 0 bis 10 Gew.-% durch Kühlen und/oder durch Desaktivierung des Katalysators mittels Säuren ab und entfernt schließlich den Katalysator.

Für die meisten der geschilderten Verfahren sind zweiwertige Bleiionen der bevorzugte Katalysator, da die Selbstkondensation des Formaldehydhydrats in Gegenwart von Verbindungen des zweiwertigen Bleis auch im neutralen oder schwach sauren Bereich mit hoher Raum/Zeit-Ausbeute und unter weitgehender Vermeidung von störenden Nebenreaktionen abläuft. Für eine Reihe von Anwendungszwecken der Formose (z.B. bei der Verwendung als Substrat für Mikroorganismen oder vor der katalytischen Hydrierung zu mehrwertigen Alkoholen) ist es erforderlich, die in den Verfahrensprodukten enthaltenen Bleiionen weitgehend zu entfernen.
Hierfür bietet sich zunächst die chemische Fällung (beispielsweise durch Zugabe von Schwefelsäure, Natriumsulfat, Natriumcarbonat, Natriumsulfid oder Kohlendioxidgas unter Druck) an. Es zeigte sich jedoch, daß die in der Formose enthaltenen Hydroxyaldehyde und Hydroxyketone eine außerordentlich starke Komplexbildungsfähigkeit mit Metallionen aufweisen, so daß die Entbleiung wäßriger Formoselösungen in großtechnischem Maßstab durch chemische Fällung nur unzureichend bzw. unter hohem technischem Aufwand möglich ist. Darüber hinaus lassen sich Formoselösungen schlecht von den ausgefällten Bleisalzen durch Filtration trennen. Außerdem müßte das Bleisalz aus wirtschaftlichen Gründen in eine lösliche und daher für die Katalyse der Formosebildung wiederverwendbare Form umgewandelt werden. Dies

Le A 18 372

- 6 -

bedingt zusätzlichen Aufwand.

Als weitere Möglichkeit bietet sich die Entfernung der Bleiionen aus den Formoselösungen mittels Ionenaustauschern an.
Der Nachteil dieses Verfahrens liegt jedoch darin, daß in
der Praxis sehr große Mengen an Austauscherharz, Spül- und
Regenerierflüssigkeit eingesetzt werden müßten, was die
Entfernung der Gesamtmenge an Blei aus Formose durch Ionenaustauscher zu einem technisch zu aufwendigen Verfahren macht.

Überraschenderweise wurde nun jedoch gefunden, daß sich die
Bleiionen aus wäßrigen Formoselösungen sehr einfach durch
kathodische elektrochemische Abscheidung entfernen lassen.
Dies hat den Vorteil, daß die bleihaltige Lösung bei der
Entbleiung nicht verdünnt wird und daß das abgeschiedene
Blei anschließend unter Umpolung der Stromquelle wieder in
Ausgangsprodukten für die Formosesynthese aufgelöst werden
kann. Es zeigte sich, daß auf diese Weise in kurzer Zeit
mehr als 90 % des in der Formose enthaltenen Bleis abgeschieden werden können. Im Hinblick auf die oben erwähnte
hohe Komplexbildungstendenz zwischen Formose und Metallionen ist dies als überraschend anzusehen, da angenommen
werden mußte, daß bei der kathodischen Abscheidung des Bleis
hohe Überspannungen auftreten würden, welche zu störenden
Nebenreaktionen (Wasserstoffabscheidung, Zersetzung der
Formose, Oxidation der Zucker) führen könnten. Unerwarteterweise ist dies jedoch nicht der Fall.

Gegenstand der vorliegenden Erfindung ist somit ein Verfahren zur Entfernung von Bleiionen aus wäßrigen Formoselösungen,
welches dadurch gekennzeichnet ist, daß man das Blei durch
kathodische, elektrochemische Abscheidung entfernt.

- 7 -

Die bei der Selbstkondensation von Formaldehydhydrat in Gegenwart von Verbindungen des zweiwertigen Bleis als Katalysator anfallenden wäßrigen Formoselösungen enthalten im allgemeinen ca. 2500 bis 4000 ppm an Bleiionen. Mit Hilfe des erfindungsgemäßen Verfahrens gelingt die Entbleiung derartiger Lösungen bis zu einer Endkonzentration an Bleiionen von ca. 10 bis 500 ppm, vorzugsweise 50 bis 300 ppm. Diese Restmenge läßt sich dann ohne großen technischen Aufwand mittels Kationenaustauschern entfernen.

Wie gefunden wurde, sind Elektrolyseverfahren mit konstanter Klemmenspannung für den beabsichtigten Zweck wenig geeignet, da während der Elektrolyse, bedingt durch die sich ständig infolge der Bleiabscheidung vergrößernde Kathodenoberfläche, die Stromstärke permanent ansteigt, wobei schnell Stromdichten erreicht werden, die zu starker Gasentwicklung führen und gleichzeitig die Lösung bis zum Sieden aufheizen. Es ist daher erfindungsgemäß bevorzugt, bei konstanter Stromstärke zu arbeiten (isocoulombische Abscheidung).

Vorzugsweise geht man dabei so vor, daß man beim Anfahren der Elektrolyse eine Anfangsstromdichte von 0,1 bis 3, besonders bevorzugt 0,3 bis 1,5, Ampere/dm$^2$ einstellt und die hierfür berechnete Stromstärke während der Elektrolyse konstant hält. Trägt man die zeitliche Abnahme der Bleiionenkonzentration in der Formoselösung für verschiedene Elektrolyseversuche mit konstanter Stromstärke in Diagrammen auf, so zeigt eine genaue Analyse der Meßwerte, daß es besonders vorteilhaft ist, das Blei in mindestens

Le A 18 372

3 Stufen abzuscheiden, wobei man in der ersten und letzten Stufe bei relativ geringen Stromdichten und in der mittleren Stufe bei relativ hohen Stromdichten arbeitet. Selbstverständlich ist es auch möglich, weitere Zwischenstufen einzubauen.

Erfindungsgemäß besonders bevorzugt ist es daher, die zu entbleiende Formoselösung hintereinander mindestens 3 Elektrolysezellen passieren zu lassen, wobei in der ersten Zelle eine Anfangsstromdichte von 0,1 bis 0,7, besonders bevorzugt 0,3 bis 0,5, Ampere/dm$^2$, in der zweiten Zelle eine Anfangsstromdichte von 0,8 bis 3,0, besonders bevorzugt 1,0 bis 1,5, Ampere/dm$^2$ und in der dritten Zelle eine Anfangsstromdichte zwischen 0,2 und 1,0, besonders bevorzugt 0,3 bis 0,8, Ampere/dm$^2$, eingestellt werden. In den einzelnen Zellen wird dabei jeweils etwa 1/3 der Gesamtmenge des abgeschiedenen Bleis entfernt. Selbstverständlich ist es, wie schon erwähnt, möglich, noch weitere Zellen mit einer jeweils zwischen den oben angegebenen Werten liegenden Anfangsstromdichte dazwischen zu schalten.

Formosen, die nach dem erfindungsgemäßen Verfahren entbleit wurden, lassen sich ohne Schwierigkeiten nach den üblichen für die Hydrierung von Zuckern bekannten Methoden hydrieren.

Die erfindungsgemäß entbleiten Formosen bzw. daraus durch gekreuzte Cannizzaro-Reaktion oder Hydrierung erhaltenen mehrwertigen Alkohole sind wertvolle Ausgangsmaterialien für eine Vielzahl anwendungstechnisch interessanter Produkte.

Beispielsweise sind sowohl die Formosen selbst als auch ihre Reduktionsprodukte (Formite) sehr gut als Kettenverlängerungsmittel bzw. Vernetzer bei der Herstellung von

Polyurethankunststoffen aus Polyisocyanaten, niedermolekularen Polyhydroxylverbindungen sowie gegebenenfalls höhermolekularen Polyhydroxylverbindungen, weiteren Kettenverlängerungsmitteln, Treibmitteln, Katalysatoren und weiteren an sich bekannten Zusatzstoffen geeignet. Durch Propoxylierung und/oder Äthoxylierung der Formosen bzw. der Formite lassen sich ferner Polyätheralkohole hoher Funktionalität gewinnen, die in hohen OH-Zahl-Bereichen für die Herstellung von harten bzw. halbharten zellförmigen Polyurethankunststoffen und bei niedrigen OH-Zahlen als Ausgangsmaterialien für hochelastische Polyurethanschaumstoffe Verwendung finden.

Durch Umsetzung von Formose oder Formit mit mehrwertigen Carbonsäuren wie z.B. Phthalsäure oder Adipinsäure nach den üblichen Verfahren der Polyesterkondensation lassen sich stark verzweigte Polyester synthetisieren, die als Zusätze zu Alkydharzen deren Härte verbessern. Selbstverständlich sind derartige Polyester ebenfalls als Ausgangskomponente zur Herstellung von Polyurethankunststoffen brauchbar.

Die erfindungsgemäß erhaltenen bleifreien Formosen bzw. Formite lassen sich auch als Feuchthaltemittel in Kosmetika und Kunststoffen verwenden. Sie können auch als Gefrierschutzmittel dienen. Ebenso ist ihre Verwendung als Kohlenhydrat-haltiges Substrat in Nährböden von Mikroorganismen möglich. Hierzu haben sich besonders diejenigen Verfahrensprodukte bewährt, die hauptsächlich aus 5 und 6 Kohlenstoffatome enthaltenden Hydroxyaldehyden und Hydroxyketonen bestehen.

Le A 18 372

Die erfindungsgemäß behandelten Formosen eignen sich infolge eines geringen Gehalts an Metallionen insbesondere auch für gezielte Methylolierungsreaktionen mittels Formaldehyd, wobei die Formosen an den zur Carbonylgruppe $\alpha$-ständigen C-Atomen durch eine Aldolkondensation mit Formaldehyd methyoliert werden. Man versetzt zu diesem Zweck die Formosen mit wäßrigem Formaldehyd bei pH = 8 - 12, vorzugsweise 9 - 11, und hält das Gemisch ca. 10 Minuten bis 12 Stunden bei 10 - 100°C, vorzugsweise 30-60°C. Als Basen sind für diesen Zweck insbesondere tertiäre Amine wie z.B. Triäthylamin, Tripropylamin oder Dimethylbenzylamin geeignet.

Die folgenden Beispiele erläutern das erfindungsgemäße Verfahren. Wenn nicht anders vermerkt, sind Mengenangaben als Gewichtsteile bzw. Gewichtsprozente zu verstehen.

In den Beispielen wird jeweils die zeitliche Abnahme des Bleigehaltes während der Elektrolyse von wäßrigen Formoselösungen sowie die zeitliche Änderung des spezifischen Widerstandes der Formoselösungen angegeben. Der "einfache Wirkungsgrad" $W_E$ ist definiert als der Quotient aus der seit Beginn der Elektrolyse abgeschiedenen Menge Blei und der aus dem Stromverbrauch berechneten theoretisch abscheidbaren Menge. Der "differentielle Wirkungsgrad" $W_D$ ist definiert als die in dem betreffenden Zeitintervall abgeschiedene Menge Blei durch in dem gleichen Intervall aufgrund des Stromverbrauchs berechneten theoretisch abscheidbaren Menge Blei.

Allgemeine Versuchsbeschreibung der Elektrolyse

In einen Chromatographiertank (12 x 7 x 19 cm) wird 1 l einer gemäß Beispiel 1 von DOS 2 639 083 hergestellten, noch nicht entsalzten 50 %igen Formoselösung mit einem Pb-Gehalt von 3390 ppm eingefüllt. Die Lösung wird mittels eines Magnetrührers langsam gerührt. In die Lösung werden im Abstand von 3,9 cm zwei Kohleplattenelektroden mit einer Oberfläche von jeweils 1,2 $dm^2$ gesenkt und eine halbe Stunde darin belassen. Man schließt eine Stromquelle und ein Voltmeter polrichtig an die Platten an und elektrolysiert die Lösung bei konstantem Strom. Gleichzeitig wird die zu jedem Meßzeitpunkt an den Klemmen bestehende Spannung registriert. Nach jeweils 15 Minuten wird der Lösung eine Probe entnommen und auf ihren Restbleigehalt untersucht. Insgesamt wurden Elektrolysen bei 7 verschiedenen Stromstärken durchgeführt mit einer Variationsbreite von 1:25.

Le A 18 372

- 12 -

Beispiel 1

Elektrolyse mit einer Konstantstromstärke von 0,24 Ampere

| Zeit (min) t | Pb-Gehalt (ppm) | spezifischer Widerstand ($\Omega \cdot$ cm) $\varsigma$ | Wirkungsgrad einfach $W_E$ | differentiell $W_D$ |
|---|---|---|---|---|
| 15 | 3165 | 555 | 0,17 | |
| 30 | 3025 | 540 | 0,32 | 0,32 |
| 45 | 2865 | 525 | 0,46 | |
| 60 | 2690 | 512 | 0,56 | 0,77 |
| 75 | 2490 | 505 | 0,63 | |
| 90 | 2290 | 502 | 0,67 | 0,92 |
| 105 | 2100 | 501 | 0,71 | |
| 120 | 1905 | 503 | 0,73 | 0,89 |
| 135 | 1720 | 500 | 0,74 | |
| 150 | 1520 | 500 | 0,765 | 0,89 |
| 165 | 1320 | 500 | 0,77 | |
| 180 | 1135 | 500 | 0,78 | 0,89 |
| 195 | 940 | 500 | 0,785 | |
| 210 | 795 | 501 | 0,775 | 0,78 |
| 225 | 670 | 503 | 0,77 | |
| 240 | 565 | 500 | 0,75 | 0,53 |
| 270 | 420 | 500 | 0,73 | 0,26 |
| 300 | 325 | 500 | 0,69 | 0,21 |

Le A 18 372

Beispiel 2

Elektrolyse mit einer Konstantstromstärke von 0,36 Ampere

| Zeit (min) t | Pb-Gehalt (ppm) | spezifischer Widerstand ($\Omega \cdot$ cm) $\varrho$ | Wirkungsgrad | |
|---|---|---|---|---|
| | | | einfach $W_E$ | differentiell $W_D$ |
| 15 | 3340 | 525 | 0,09 | |
| 30 | 3230 | 516 | 0,23 | 0,23 |
| 45 | 2960 | 495 | 0,36 | |
| 60 | 2700 | 480 | 0,475 | 0,76 |
| 75 | 2430 | 470 | 0,54 | |
| 90 | 2180 | 455 | 0,58 | 0,74 |
| 105 | 1920 | 449 | 0,61 | |
| 120 | 1660 | 445 | 0,63 | 0,75 |
| 135 | 1400 | 444 | 0,645 | |
| 150 | 1140 | 440 | 0,64 | 0,75 |
| 165 | 960 | 441 | 0,635 | |
| 180 | 720 | 440 | 0,63 | 0,60 |
| 195 | 580 | 440 | 0,62 | |
| 200 | 480 | 439 | 0,595 | 0,35 |
| 225 | 390 | 438 | 0,575 | |
| 240 | 350 | 439 | 0,54 | 0,18 |

Le A 18 372

- 14 -

Beispiel 3

Elektrolyse mit einer Konstantstromstärke von 0,6 Ampere

| Zeit (min) t | Pb-Gehalt (ppm) | spezifischer Widerstand ($\Omega \cdot cm$) | Wirkungsgrad einfach $W_E$ | differentiell $W_D$ |
|---|---|---|---|---|
| 15 | 3160 | 452 | 0,13 | |
| 30 | 2885 | 430 | 0,30 | 0,30 |
| 45 | 2510 | 412 | 0,415 | |
| 60 | 2215 | 397 | 0,49 | 0,58 |
| 75 | 1840 | 385 | 0,52 | |
| 90 | 1495 | 377 | 0,535 | 0,63 |
| 105 | 1110 | 370 | 0,54 | |
| 120 | 865 | 360 | 0,535 | 0,55 |
| 135 | 660 | 357 | 0,51 | |
| 150 | 555 | 360 | 0,48 | 0,26 |
| 165 | 460 | 359 | 0,45 | |
| 180 | 405 | 358 | 0,43 | 0,13 |
| 195 | 395 | 354 | 0,40 | |
| 210 | 345 | 356 | 0,38 | - |
| 225 | 320 | 355 | 0,36 | |
| 240 | 305 | 355 | 0,335 | - |

Beispiel 4

Elektrolyse mit einer Konstantstromstärke von 1,2 Ampere

| Zeit (min) t | Pb-Gehalt (ppm) | spezifischer Widerstand ($\Omega \cdot cm$) $\mathcal{G}$ | Wirkungsgrad einfach $W_E$ | differentiell $W_D$ |
|---|---|---|---|---|
| 15 | 3310 | 370 | 0,03 | |
| 30 | 3120 | 355 | 0,10 | 0,10 |
| 45 | 2600 | 335 | 0,20 | |
| 60 | 2100 | 315 | 0,26 | 0,445 |
| 75 | 1600 | 295 | 0,32 | |
| 90 | 1100 | 275 | 0,33 | 0,44 |
| 105 | 900 | 270 | 0,31 | |
| 120 | 780 | 270 | 0,28 | 0,14 |
| 135 | 660 | 270 | 0,26 | |
| 150 | 530 | 270 | 0,24 | 0,11 |
| 165 | 420 | 268 | 0,225 | |
| 180 | 310 | 266 | 0,22 | 0,095 |
| 195 | 250 | 265 | 0,21 | |
| 210 | 180 | 265 | 0,20 | 0,06 |
| 225 | 160 | 263 | 0,19 | |
| 240 | 125 | 263 | 0,18 | 0,02 |

Le A 18 372

Beispiel 5

Elektrolyse mit einer Konstantstromstärke von 1,8 Ampere

| Zeit (min) t | Pb-Gehalt (ppm) | spezifischer Widerstand ($\Omega \cdot$ cm) $\varrho$ | Wirkungsgrad einfach $W_E$ | differentiell $W_D$ |
|---|---|---|---|---|
| 15 | 3100 | 325 | 0,02 | |
| 30 | 2765 | 300 | 0,08 | 0,12 |
| 45 | 2220 | 270 | 0,16 | |
| 60 | 1650 | 245 | 0,205 | 0,33 |
| 75 | 1140 | 230 | 0,21 | |
| 90 | 765 | 228 | 0,205 | 0,27 |
| 105 | 540 | 228 | 0,20 | |
| 120 | 420 | 227 | 0,18 | 0,10 |
| 135 | 320 | 226 | 0,165 | |
| 150 | 240 | 225 | 0,16 | 0,055 |
| 165 | 160 | 225 | 0,15 | |
| 180 | 100 | 225 | 0,145 | 0,04 |
| 195 | 90 | 224 | 0,14 | |
| 210 | 80 | 222 | 0,13 | - |
| 225 | 70 | 220 | 0,125 | |
| 240 | 60 | 220 | 0,12 | - |

Beispiel 6

Elektrolyse mit einer Konstantstromstärke von 3,6 Ampere

| Zeit (min) t | Pb-Gehalt (ppm) | spezifischer Widerstand ($\Lambda \cdot$ cm) $\rho$ | Wirkungsgrad einfach $W_E$ | differentiell $W_D$ |
|---|---|---|---|---|
| 15 | 3180 | 300 | 0,02 | |
| 30 | 2745 | 255 | 0,07 | 0,06 |
| 45 | 1800 | 205 | 0,14 | |
| 60 | 1065 | 170 | 0,16 | 0,25 |
| 75 | 800 | 150 | 0,15 | |
| 90 | 520 | - | 0,12 | 0,08 |

nach 100 Minuten beginnt die Lösung zu kochen

Beispiel 7

Elektrolyse mit einer Konstantstromstärke von 5,4 Ampere

| Zeit (min) t | Pb-Gehalt (ppm) | spezifischer Widerstand ($\Lambda \cdot$ cm) $\rho$ | Wirkungsgrad einfach $W_E$ | differentiell $W_D$ |
|---|---|---|---|---|
| 15 | 3000 | 255 | 0,02 | 0,03 |
| 30 | 1930 | 130 | 0,12 | 0,13 |
| 45 | 1170 | - | 0,13 | 0,135 |
| 60 | 640 | - | 0,125 | 0,14 |
| 75 | 340 | - | 0,11 | 0,06 |

nach 85 Minuten beginnt die Lösung zu kochen

Le A 18 372

Beispiel 8

Dieses Beispiel zeigt, daß sich das erfindungsgemäße Verfahren optimieren läßt, wenn man mehrere Elektrolyseschritte mit unterschiedlicher konstanter Stromstärke hintereinander setzt.

Bei einer Verfahrensweise unter Einhalten einer kleinen konstanten Stromstärke ist zwar der Wirkungsgrad sehr hoch (Beispiel 1), jedoch dauert die Elektrolyse bis zu einem vorgegebenen Restbleigehalt relativ lange. Bei der Verfahrensweise nach Beispiel 7 unter Einhalten einer hohen Stromstärke sind die benötigten Zeiten zwar relativ kurz, dafür ist jedoch der Wirkungsgrad schlecht.

Das nachfolgend beschriebene Beispiel zeigt die erfindungsgemäß bevorzugte Verfahrenweise auf. Nach dieser Verfahrensweise elektrolysiert man nacheinander mit unterschiedlichen, aber jeweils konstanten Stromstärken. Es werden drei Apparaturen gleicher Art, wie sie unter "allgemeine Arbeitsweise" beschrieben sind, eingesetzt. In der ersten Apparatur wird mit einer konstanten Stromstärke von 0,36 Amp, in der zweiten mit 1,2 Amp und in der dritten mit 0,6 Amp elektrolysiert. 1 Liter Formoselösung wie in Beispiel 1 wird dabei innerhalb von 42 Minuten in der ersten Apparatur bis auf 2200 ppm, in der zweiten Apparatur innerhalb von 21 Minuten bis auf 1600 ppm und in der dritten Apparatur in 72 Minuten bis auf 500 ppm entbleit (selbstverständlich kann auch bis zu einem geringeren Bleigehalt in der dritten Zelle gefahren werden, doch wurde zwecks besserer Vergleichbarkeit die Elektrolyse schon bei 500 ppm abgebrochen).

Le A 18 372

In diesem Zusammenhang sei darauf hingewiesen, daß man selbstverständlich das Elektrolyseverfahren auch kontinuierlich durchführen kann, indem man nicht wie in diesem Beispiel gezeigt die Lösung jeweils von einer Apparatur in die nächste umfüllt (diskontinuierlich), sondern stattdessen die Lösung durch hintereinandergeschaltete Zellen strömen läßt. Notwendige und hinreichende Voraussetzung für einen solchen kontinuierlichen Betrieb ist, die Zellenvolumina und die Elektrodenoberflächen auf die Fließgeschwindigkeit abzustimmen; dabei gilt, daß sich die Volumina der einzelnen Zellen umgekehrt zueinander verhalten, wie die Elektrolysestromstärken in den Zellen und die Elektrodenoberflächen den Stromstärken proportional sind.

Der quantitative Vergleich zwischen den Beispielen 1, 7 und 8 hinsichtlich ihres Wirkungsgrades bezüglich der Bleiabscheidung einerseits und der dafür benötigten Elektrolysierzeiten andererseits zeigt, daß die Verfahrensweise nach Beispiel 8 gegenüber jener von Beispiel 1 und 7 optimiert ist, wenn man postuliert, daß eine Verfahrensweise dann als optimal zu bezeichnen ist, wenn das Produkt P aus Wirkungsgrad und Abscheidegeschwindigkeit zu jedem Zeitpunkt der Elektrolyse maximal ist:

$$P = W_D \cdot \frac{\text{Abscheidemenge}}{\text{Zeitintervall}}$$

Dies ist, wie eine Analyse der Meßdaten von Beispiel 1 bis 7 zeigt, bei der Arbeitsweise von Beispiel 8 der Fall. Auch die Gesamtwirkung ist in Beispiel 8 besser.

Le A 18 372

- 20 -

Vergleich der Gesamtwirkung $\omega$ $\left(\omega = \int_0^t W_E \cdot dt\right)$ der

Beispiele 1, 7 und 8 und der für einen Restbleigehalt von
500 ppm benötigten Elektrolysezeiten:

| Beispiel 7 | —— | Beispiel 8 |
|---|---|---|
| $t_7 : t_8$ | = | 1:2 |
| $\omega_7 : \omega_8$ | = | 1:12 |

| Beispiel 1 | —— | Beispiel 8 |
|---|---|---|
| $t_1 : t_8$ | = | 1,8:1 |
| $\omega_1 : \omega_8$ | = | 2,7:1 |

Somit ergibt sich, daß die Verfahrensweise nach Beispiel 8
12 mal effektiver ist als die Verfahrensweise nach Beispiel 7,
und nur ca. 2 mal langsamer; gegenüber Beispiel 1/ca. 3mal nur weniger
effektiv aber ca. 2mal schneller.

Durch eine weitere Erhöhung der Anzahl der Elektrolysestufen
ließe sich eine noch etwas effektivere Arbeitsweise ermöglichen, doch steigt dann wieder der apparative Aufwand
stark an.

Durch Umpolen der Stromquelle nach Auswechseln der als Anode
verwendeten Kohleelektrode gegen eine Stabelektrode (vorzugsweise aus Silber oder Kupfer) gelingt es, das zuvor an
der Kathode abgeschiedene Blei anodisch wieder aufzulösen.
Vorzugsweise führt man diese anodische Auflösung des Bleis
in einer wäßrigen Formoselösung aus, welche später als Co-

Le A 18 372

Katalysator für die Formoseherstellung eingesetzt werden soll; man erhält auf diese Weise eine sowohl katalytisch als auch cokatalytisch besonders wirksame bleihaltige Formoselösung mit einem Bleigehalt von 30 000 ppm und mehr. Derartige Co-Katalysatorlösungen sind für die Formosesynthese besonders bevorzugt, da das Blei in der wäßrigen Formoselösung ohne Fremdanion komplexiert vorliegt, was an der tiefgrünen bis schwarzgrünen Farbe einer solchen bleihaltigen Formoselösung erkennbar ist.

- 22 -

Patentansprüche

1) Verfahren zur Entfernung von Bleiionen aus wäßrigen
Formoselösungen, dadurch gekennzeichnet, daß man das
Blei durch kathodische elektrochemische Abscheidung
entfernt.

2) Verfahren nach Anspruch 1, dadurch gekennzeichnet, daß
die Abscheidung bei konstanter Stromstärke erfolgt.

3) Verfahren nach Anspruch 1 oder 2, dadurch gekennzeichnet, daß mit einer Anfangsstromdichte von 0,1 bis 3
Ampere/dm$^2$ elektrolysiert wird.

4) Verfahren nach Anspruch 3, dadurch gekennzeichnet, daß
mit einer Anfangsstromdichte von 0,3 bis 1,5 Ampere/dm$^2$
elektrolysiert wird.

5) Verfahren nach Ansprüchen 1 bis 4, dadurch gekennzeichnet, daß man das Blei in mindestens 3 Stufen abscheidet,
wobei in der ersten und letzten Stufe niedrigere Stromdichten eingehalten werden als in den mittleren Stufen.

6) Verfahren nach Anspruch 5, dadurch gekennzeichnet, daß
man in den einzelnen Stufen isocoulombisch arbeitet.

7) Verfahren nach Anspruch 6, dadurch gekennzeichnet, daß
man in der ersten Stufe eine Anfangsstromdichte von 0,1
bis 0,7 Ampere/dm$^2$, in der mittleren Stufe eine solche von
0,8 bis 3,0 und in der letzten Stufe eine solche von 0,2
bis 1,0 Ampere/dm$^2$ einhält.

Le A 18 372

8. Verwendung der gemäß Anspruch 1 bis 7 entbleiten Formosen als Ausgangskomponenten für die Herstellung von gegebenenfalls zellförmigen Polyurethankunststoffen.

9. Verwendung der gemäß Anspruch 1 bis 7 entbleiten Formosen als Ausgangskomponenten für die Herstellung von Polyester- oder Polyätherpolyolen.

10. Verwendung der gemäß Anspruch 1 bis 7 entbleiten Formosen für die Herstellung gezielt $\alpha$-methylolierter Formosen.

| | **EUROPÄISCHER RECHERCHENBERICHT** | Nummer der Anmeldung |
|---|---|---|
| Europäisches Patentamt | | EP 78 10 06|

| **EINSCHLÄGIGE DOKUMENTE** | | | **KLASSIFIKATION DER ANMELDUNG (Int. Cl.²)** |
|---|---|---|---|
| Kategorie | Kennzeichnung des Dokuments mit Angabe, soweit erforderlich, der maßgeblichen Teile | betrifft Anspruch | |
| DA | <u>US - A - 2 272 378</u> (HERCULES POWDER)<br><br>* Seite 2, rechte Spalte, Abschnitt 4 *<br><br>-- | 1 | C 07 H  3/04<br>       3/02<br>C 07 C 49/17<br>       47/19<br>       47/10<br>       31/18<br>C 08 G 18/32<br>       63/12<br>       65/28 //<br>C 08 G 18/42<br>       18/48 |
| A | <u>US - A - 3 457 152</u> (J.N. MALONEY)<br><br>* Zusammenfassung; Spalte 4, Zeilen 21-26 *<br><br>-- | 1 | |
| P | <u>DE - A - 2 639 084</u> (BAYER)<br><br>* Seite 20; Abschnitt 2 *<br><br>-- | 1 | **RECHERCHIERTE SACHGEBIETE (Int. Cl.²)**<br><br>C 07 H  3/04<br>       3/02<br>C 07 C 49/17<br>       49/19<br>       47/10<br>       31/18 |
| E | <u>DE - A - 2 714 084</u> (BAYER)<br><br>* Seite 15, Abschnitt 2 *<br><br>-- | 1 | |
| E | <u>DE - A - 2 714 104</u> (BAYER)<br><br>* Seite 14, zwei letzten Zeilen; Seite 15, erster Abschnitt; Seite 20, letzter Abschnitt; Seite 21, erster Abschnitt *<br><br>---- | 1 | |

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 28-11-1978 | STOOS |

EPA form 1503.1  06.78